# EUROPEAN PATENT SPECIFICATION

(11) **EP 0 925 310 B1**
(45) Date of publication and mention of the grant of the patent: **16.08.2000**
(21) Application number: 97939097.8
(22) Date of filing: 10.09.1997
(51) Int. Cl.: C08B 15/00, C08B 37/04, A61K 31/715

(54) **USE OF SULFATED POLYSACCHARIDES FOR THE TREATMENT OF A CHRONIC WOUND**
VERWENDUNG VON SULFATIERTEN POLYSACCHARIDEN ZUR BEHANDLUNG EINER CHRONISCHER VERLETZUNG
UTILISATION DE POLYSACCHARIDES SULFATES DANS LE TRAITEMENT DE PLAIES CHRONIQUES

(30) Priority: 11.09.1996 GB 9618958
(43) Date of publication of application: 30.06.1999
(73) Proprietor: JOHNSON & JOHNSON MEDICAL LIMITED., Edinburgh EH2 4NH (GB)
(72) Inventor: GRADY, Michael, William, West Yorkshire LS29 3RX (GB); DOYLE, Peter, John, West Yorkshie LS29 6PP (GB); SINCLAIR, Laura, West Yorkshire LS29 9DR (GB); HOUSTON, Paul, Edinburgh EH12 8NP (GB)
(74) Representative: James, Anthony Christopher W.P.
(86) International application number: GB9702477
(87) International publication number: WO9811141

(56) References cited:
- EP-A- 0 140 596
- DE-A- 2 546 699
- FR-A- 1 056 772
- US-A- 3 709 877
- US-A- 4 879 282
- RICHARD G. SCHWEIGER ET AL.: "Polysaccharide Sulfates." CARBOHYDRATE RESEARCH., vol. 21, 1972, AMSTERDAM NL, pages 275-281, XP002049669
- KURT MAURER ET AL.: "Die Synthese heparinwirksamer Verbindungen aus carboxylierter Cellulose." CHEMISCHE BERICHTE., vol. 80, no. 1, 1947, WEINHEIM DE, pages 179-187, XP002049670

## Description

The present invention relates to the use of sulfated polysaccharides obtained by sulfation of cellulose derivatives or polyanionic polysaccharides and uses thereof in pharmaceutical and wound treatment compositions.

Sulfated saccharide and polysaccharide derivatives, in which one or more hydroxyl groups on individual saccharide residues are replaced by sulfate groups, are known. Such sulfated saccharides can occur naturally, as in the case of the sulfated glucosaminoglycans such as heparan sulfate and chondroitin sulfate. Other sulfated saccharides, oligosaccharides and polysaccharides have been prepared by sulfation of naturally occurring saccharides, oligosaccharides or polysaccharides.

A known synthetic sulfated saccharide derivative is sucrose octasulfate, obtained by sulfation of sucrose. The insoluble aluminum salt of sucrose octasulfate (known as sucralfate) has been proposed for a number of medicinal uses, including the enhancement of wound healing in EP-A-0230023.

US-A-2697093 describes the sulfation of cellulose, inulin, starch or dextrin with sulfur trioxide-tertiary amine reagents. The resulting sulfated materials are stated to be useful as thickeners for paste, adhesives, and additives for muds used in drilling oil wells.

Certain sulfated polysaccharides, and in particular the sulfated glucosaminoglycans, are used in medicine as anticoagulants. In addition, heparin, a highly polydisperse copolymer of 1-4 linked glucosamine and uronic acid residues, plays an important role in the control of blood coagulation. Heparin binds the serine protease inhibitor antithrombin producing a complex which accelerates the proteolysis of the enzyme responsible for coagulation. The anticoagulant properties of heparin and its analogs have been known for some time and have been usefully applied in the biomedical field. The preparation of heparin and sulfated glucosaminoglycans from natural sources is expensive, and therefore an alternative is desirable.

It has now been found that sulfation of cellulose derivatives such as oxidised regenerated cellulose (ORC) or polyanionic polysaccharides such as sodium alginate results in sulfated polysaccharide materials that provide unexpected advantages as wound healing materials, and further exhibit anticoagulant properties.

The term "cellulose derivative" encompasses any biologically acceptable ester, ether, hydrolysis product or oxidation product of cellulose. Preferred cellulose derivatives include carboxymethyl cellulose, hydroxyethyl cellulose, cellulose acetate and, especially, oxidized cellulose derivatives such as oxidized regenerated cellulose (ORC), or the oxidized cellulose ethers and esters described and claimed in the provisional US patent application USSN 60/020,758 assigned to the same assignee as the present application and filed on 28th June 1996 and entitled "Bioresorbable Medical Devices from Oxidized Cellulose Derivatives". Preferred materials are obtained by oxidation of hydroxyethyl cellulose, carboxymethyl cellulose or cellulose acetate with dinitrogen tetroxide. The most preferred material is oxidized regenerated cellulose (ORC).

The term "polyanionic polysaccharide" encompasses any biologically acceptable polysaccharide in which a plurality of the saccharide residues in each polysaccharide molecule comprise an anionic group such as carboxylate, or the complementary acid or salt of such an anionic group. It will be appreciated that some of the cellulose derivatives defined above will also be polyanionic polysaccharides. Preferred polyanionic polysaccharides include alginates, pectins, hyaluronic acid, oxidized starch and oxidized cellulose derivatives as described above. Most preferably, the polyanionic polysaccharide is an alginate. Naturally occurring sulfated polyanionic polysaccharides such a chondroitin sulfate are not included within the scope of the present invention, which relates only to synthetic (i.e. chemically sulfated) polysaccharide derivatives.

Oxidised regenerated cellulose (ORC) is a well known cellulose derivative that is prepared from cellulose as described in US-A-3122479. Oxidation of the primary hydroxyl groups of cellulose to carboxylate groups results in a polyanionic cellulose derivative, namely ORC. ORC is known and used in the biomedical field as a haemostatic agent, as described in US-A-2517772. It is commercially available in the form of a fabric under the registered trade mark Surgicel (Johnson & Johnson Medical, Inc.), and as a haemostatic wound treatment composition under the registered trade mark Interceed (Johnson & Johnson Medical, Inc.). It has been found that ORC is both haemostatic and fully bioabsorbable, and thus provides important advantages as a wound dressing material.

The term "alginate" used herein encompasses alginic acid, soluble salts thereof such as sodium alginate, and insoluble salts thereof such as calcium alginate. Preferably, the alginate is calcium alginate, which is available for use in wound dressings under the Registered Trade Marks Instat (Johnson & Johnson Medical, Inc.) and Kaltostat (E.R. Squibb & Sons, Inc.). Preferably, the alginate has a molecular weight in the range 50,000 to 400,000 and a mannuronic acid/guluronic acid ratio of about 0.45.

The sulfated polysaccharides used in the present invention differ from the non-sulfated base materials in that at least some of the hydroxyl groups on the saccharide residues of the polysaccharides have been converted to sulfate groups. Preferably, an average of at least 0.1 hydroxyl groups on each saccharide residue have been converted to sulfate groups. More preferably, at least 1 hydroxyl group, on average, on each saccharide residue has been converted to sulfate groups, and most preferably from 3 to 4 hydroxyl groups on each saccharide residue have been so converted.

The sulfated polysaccharides can exist in the free acid form, but will more normally be used in the form of a salt or hydrate. The salt may be a salt with an alkali metal cation such as sodium or potassium, or may be a salt formed with polyvalent ions such as calcium or aluminum (by analogy with sucralfate), or may be a salt formed with complex cations such as ammonium or alkylammonium. Of course, the sulfated polysaccharide or its soluble salts may be stored or used in the form of a buffered aqueous solution. In any case, the term "sulfated polysaccharide" in the present specification encompasses all such salts and hydrates.

The sulfated polysaccharides can be made with substantially any molecular weight from 500 to 5,000,000, depending on the molecular weight of the starting material. Low molecular weight sulfated polysaccharides generally have alkali metal salts that are soluble in water, and accordingly such materials having molecular weights in the range 1,000 - 50,000 are preferred for some applications. These materials preferably have solubility of at least 10g/l, more preferably at least 25g/l in water at pH7.

When an insoluble sulfated material is desired, the base polysaccharide preferably has an average molecular weight in the range 50,000 - 250,000, more preferably 50,000 - 150,000. Such sulfated polysaccharides and/or their salts are usually Substantially insoluble in water. For example, insoluble sulfated ORC can be prepared from commercially available fibrous ORC in the form of a woven, non-woven or knitted fabric, thereby providing a sulfated ORC in the form of a fabric.

The present invention provides a pharmaceutical composition comprising a sulfated cellulose derivative and/or a sulfated anionic polysaccharide. The term "pharmaceutical composition" includes wound treatment compositions and wound dressings and implants, as well as topical, oral or parenteral medicinal compositions.

It has been found, surprisingly, that the sulfated polysaccharides have an exceptional ability to bind to matrix metalloproteinases (MMP's). Such matrix metalloproteinases are implicated in a number of medical conditions, including chronic wounds such as decubitis ulcers. This is because the balance between matrix deposition and tissue turnover, which in turn may depend on the balance between proteolytic enzymes and their inhibitors, is fundamental to wound healing and to certain other medical conditions. Chronic wound fluids have been shown to contain elevated levels of MMP2 (Gelatinase A) and MMP9 (Gelatinase B).

Accordingly, the present invention further provides the use of a sulfated polysaccharide for the preparation of a composition for the treatment of medical conditions mediated by a matrix metalloproteinase. Preferably, the medical condition is a wound, especially a chronic wound or ulcer.

This aspect of the invention further encompasses the use of a pharmaceutical composition containing the sulfated polysaccharide, preferably together with conventional pharmaceutical excipients, for the treatment of a chronic wound such as a venous ulcer or a decubitis ulcer in a mammal by the topical or systemic administration thereof.

Preferably, the sulfated oligosaccharide is applied topically as or in a wound dressing composition. Preferably, the sulfated polysaccharide is applied in an ointment containing 0.1%-10% w/w of the sulfated polysaccharide together with conventional excipients such as water and a gelling agent.

The pharmaceutical compositions according to the present invention may further comprise conventional pharmaceutical excipients and/or other active agents. They may be adapted for topical, oral or parenteral administration.

The sulfated anionic polysaccharides can be prepared by sulfation of a cellulose derivative or a polyanionic polysaccharide with sulfur trioxide-tertiary amine complexes or sulfur trioxidepyridine complexes in a suitable inert polar solvent, such as N-methyl pyrrolidone. The degree of sulfation can be controlled by adjusting the molar ratio of polysaccharide to sulfur trioxide, since the reaction is essentially quantitative. The extend of sulfation can be monitored by nmr and elemental analysis, and in particular by Infrared spectroscopy.

If a low molecular weight, soluble, sulfated polysaccharide derivative is desired, then the sulfation is preferably carried out on a low molecular weight, soluble oligosaccharide. For example, the sulfation can be carried out on ORC oligosaccharides obtained as described in the present applicant's co-pending United Kingdom patent application no. 9613683.3 entitled "Oxidised Oligosaccharides". Briefly, the ORC oligosaccharides are prepared by treating commercially available ORC with 6M sodium hydroxide solution at 37°C for 45 minutes, followed by filtration. The filtrate containing the ORC oligosaccharides in solution is subjected to neutralisation and dialysis to remove fragments and impurities having molecular weight below 1000, and is then freeze-dried.

Specific embodiments of the invention will now be described further, by way of example, with reference to the accompanying drawings, in which:
Figure 1 shows the measured amount of MMP binding for a sulfated ORC derivative according to the present invention, compared with ORC itself and also compared with a collagen control; and
Figure 2 shows the measured amount of MMP2 and MMP9 binding for sulfated alginate/collagen sponges compared with a pure collagen sponge and non-sulfated alginate/collagen sponges.

### Example 1

Milled ORC (Interceed®, lOg) was activated by slurrying in 100ml deionised water with stirring for 40 minutes at 40°C. The slurry was filtered using a Buchner apparatus, and the resultant cake was resuspended in 100ml glacial acetic acid and stirred for a further 30 minutes at 4°C to remove any excess water. The slurry was again filtered and washed extensively with N-methyl pyrrolidone (NMP) until all the acetic acid was removed.

Pyridine sulfur trioxide (40g) was dissolved in 40ml N-methyl pyrrolidone, cooled to 4°C, and slowly added to the activated ORC. The slurry was stirred at 4°C for 2 hours, and the pH was then adjusted to 5.6 by the careful addition of cold 2M NaOH. The neutralised slurry was poured into three volumes of methanol and the precipitate collected. The sulfated material was washed with several portions of methanol to remove the N-methyl pyrrolidone before being washed with distilled water to remove unbound sodium sulfate. The material was frozen and freeze-dried.

FT-IR analysis of the material revealed that the all of the primary hydroxyl groups and one or more of the secondary hydroxyl groups had been sulfated. The product is the insoluble sodium salt of the sulfated ORC.

### Example 2

The procedure of Example 1 was repeated on a lOg sample of Interceed® ORC fabric, resulting in a sulfated ORC fabric product.

### Example 3

A composite material consisting of a complex between the sulfated ORC according to the present invention and collagen was prepared as follows.

Sulfated ORC fibres (0.875g) prepared as in Example 1 were suspended in 10ml distilled 0.06M acetic acid and stirred until fully dispersed. Bovine collagen, which was fully limed, freeze-dried and milled to lmm fibers (1.625g) was slurried in 250ml 0.05M acetic acid and homogenised for 15 seconds on a Waring Blendor. The suspension of sulfated ORC was added to the collagen slurry, and the mixture was homogenised for a further 15 seconds. The slurry was crosslinked by the addition of 0.0325ml of HMDI, followed by homogenisation for 2 x 15 seconds. The slurry was degassed under vacuum, poured into 9cm by 9cm petri dishes, frozen and freeze-dried. The resultant sponges were sterilised with gamma-irradiation.

### Example 4

A sulfated calcium alginate was prepared from Alginic acid supplied by Sigma Chemical Company (A7003). A sample of this material (10g) was sulfated as described in Example 1.

### Example 5

A composite material consisting of a complex between the sulfated alginate obtained in Example 4 and collagen was prepared by the procedure described in Example 3, using milled sulfated alginate fibers in place of the sulfated ORC fibers.

### Procedure 1: Effect of Sulfation on Matrix Metalloproteinase Binding by ORC

The effect of sulfation of ORC on matrix metalloproteinase (MMP) binding was assessed as follows.

Sulfated ORC was prepared by the procedure described in Example 2. An ORC-free collagen sponge was prepared for comparison purposes. A sample of Surgicel® ORC fabric was also prepared for comparison.

Briefly 50mg of each material was placed in a 15ml plastic beaker containing 2.5ml of an acute wound fluid diluted to 1:50 in a proteolysis buffer (50mM tris/HCL pH7.8, 50mM CaCl₂, 0.5M NaCl) and incubated at 37°C on a shaking water bath for 3 hours. Acute wound fluid contains various proteinases, including matrix metalloproteinases and many of these enzymes will preferentially bind to various dressing materials. The excess fluid absorbed by each material was mechanically expressed using a metal spatula and discarded. The remaining dressings were placed into pre-packed 2ml syringes (each syringe contained 0.5ml volume of 2.5mm glass beads). 4ml of proteolysis buffer was forced through the syringe in 1ml aliquots which were discarded. At this washing stage all of the unbound proteinases and proteinaseses which were only weakly bound to the dressing material had been removed from the dressing leaving the more tightly bound forms. The buffer rinsed dressing were then removed to another 15ml plastic beaker. 1ml of non-denaturing sample buffer (6.3ml 0.05M tris/HCL pH6.8, 2.5ml glycerol, 0.5g SDS, 16.2ml water and bromophenol blue) was added to each sample which were placed on an orbital shakier at setting six for 2 hours. The sample buffer detaches the tightly bound proteinases from the materials which are then present in the sample buffer itself. After this time 20 microliters of sample buffer was taken from each container and subjected to gelatin substrate SDS-polyacrylamide gel electrophoresis (zymography) as described by Heussen C. and Dowdle E.B. in Anal. Biochem. 102:196-202 (1980).

The area of the individual zones of clearance on the gels, which are due to proteinase activity, were accurately measured by the Optilab system. This was achieved by repeating each binding experiment (n=3) and analysing the results statistically by the Students T test, where P ≤ 0.05. Analysis was against controls of pure collagen.

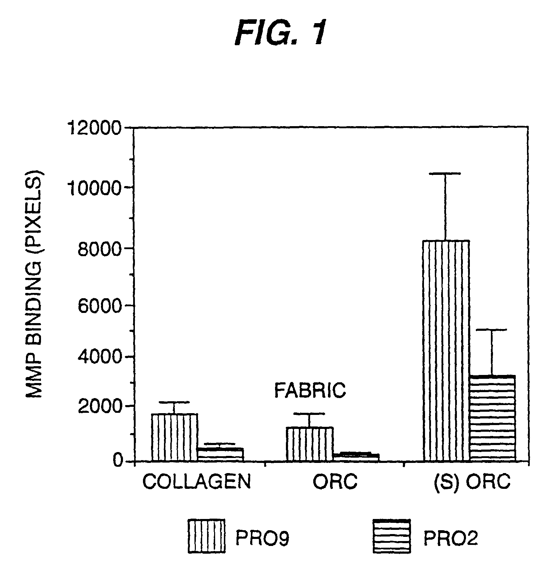

The results shown in Figure 1 demonstrate a surprising synergistic improvement in MMP binding for the sulfated ORC. In Figure 1, Pro2 and Pro9 are the proenzyme forms of MMP2 and MMP9. Both MMP2 and MMP9 can exist as active an proenzyme forms. The molecular weight of the proMMP9 or pro9 is 92kDa. MMP activation is a two step process, a conformational change occurs in the proenzyme, followed by removal of the propeptide by a series of autocatalytic changes. The molecular weight of the resultant active forms being 86kDa for ActMMP9 and 66kDa for ActMMp2, sometimes called ACT9 and ACT2 for short.

### Procedure 2: Effect of Sulfation of Alginate on Matrix Metalloproteinase Binding by Collagen-Alginate Complexes:

Procedure 1 was repeated on the following samples: a pure collagen sponge (comparative experiment), collagen/calcium alginate sponges containing 5% and 20% w/w of alginate (comparative experiments identified as 5% ALG and 20% ALG), and collagen/sulfated calcium alginate sponges containing 5%, 10% and 20% w/w of sulfated calcium alginate, prepared as described in Example 5 and identified as 5% SALG, 10% SALG and 20% SALG.

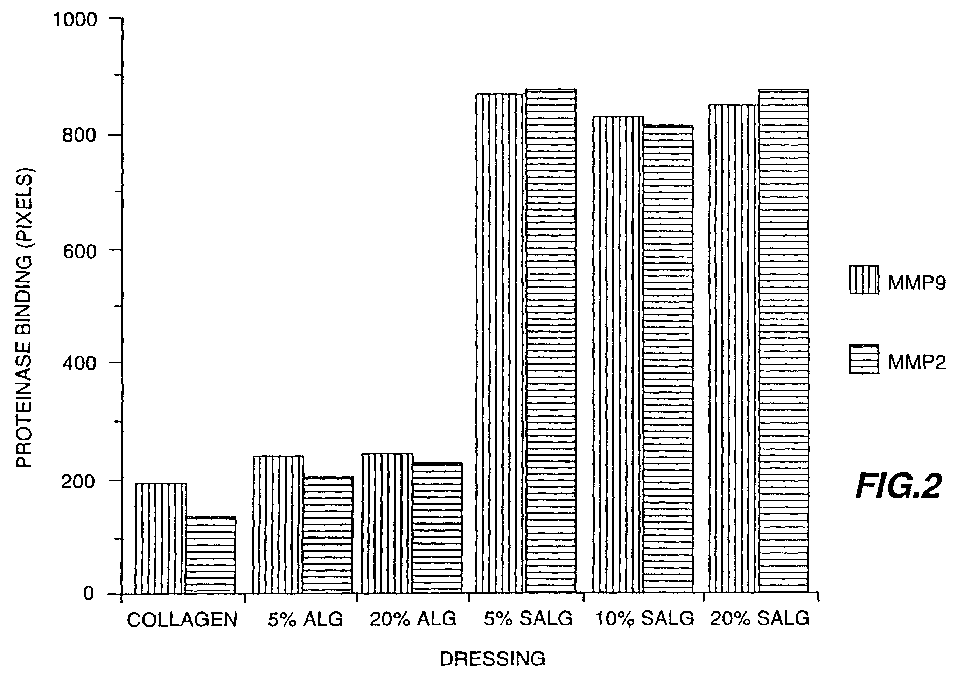

The results are shown in Figure 2. It can be seen that the collagen/sulfated alginate sponges exhibit much stronger binding to both MMP2 (Gelatinase A) and MMP9 (Gelatinase B) than the comparative sponges.

The above described specific embodiments of the present invention are intended solely for the purpose of illustration. Many other embodiments falling within the scope of the accompanying claims will be apparent to the skilled reader.

## Claims

1. Use of a chemically sulfated polysaccharide selected from the group consisting of sulfated cellulose derivatives and chemically sulfated polyanionic polysaccharides for the preparation of a composition for the treatment of a chronic wound.

2. Use according to claim 1, wherein the sulfated polysaccharide is selected from the group consisting of sulfated hydroxyethyl cellulose, sulfated carboxymethyl cellulose and sulfated oxidized regenerated cellulose.

3. Use according to claim 2, wherein the sulfated polysaccharide is sulfated oxidized regenerated cellulose.

4. Use according to claim 1, wherein the sulfated polysaccharide is selected from the group consisting of sulfated alginates, sulfated pectins and sulfated hyaluronic acid.

5. Use according to claim 4, wherein the sulfated polysaccharide is a sulfated alginate.

6. Use according to any preceding claim, wherein the sulfated polysaccharide comprises an average of at least 0.1 sulfate groups for each saccharide residue of the polysaccharide.

7. Use according to claim 6, wherein the sulfated polysaccharide comprises an average of at least 1 sulfate group for each saccharide residue of the polysaccharide.

8. Use according to any preceding claim, wherein the sulfated polysaccharide has an average molecular weight in the range 25,000 to 250,000.

9. Use according to any preceding claim, wherein the sulfated polysaccharide is in the form of a woven, non-woven or knitted fabric.

10. Use according to any preceding claim, wherein the sulfated polysaccharide is in the form of a solid complex with collagen.

11. Use according to any preceding claim, wherein the sulfated polysaccharide is soluble in water to an extent of at least 10 g/l at 25°C.

## Patentansprüche

1. Verwendung eines chemisch sulfatierten Polysaccharides, das aus der Gruppe ausgewählt ist, die aus sulfatierten Zellulosederivaten und chemisch sulfatierten polyanionischen Polysacchariden besteht, zur Herstellung einer Zusammensetzung für die Behandlung einer chronischen Wunde.

2. Verwendung nach Anspruch 1, bei der das sulfatierte Polysaccharid aus der Gruppe ausgewählt ist, die aus sulfatierter Hydroxyethylzellulose, sulfatierter Carboxymethylzellulose und sulfatierter oxidierter regenierierter Zellulose besteht.

3. Verwendung nach Anspruch 2, bei der das sulfatierte Polysaccharid sulfatierte oxidierte regenerierte Zellulose ist.

4. Verwendung nach Anspruch 1, bei der das sulfatierte Polysaccharid aus der Gruppe ausgewählt ist, die aus sulfatierten Alginaten, sulfatierten Pectinen und sulfatierter Hyaluronsäure besteht.

5. Verwendung nach Anspruch 4, bei der das sulfatierte Polysaccharid ein sulfatiertes Alginat ist.

6. Verwendung nach einem der vorangehenden Ansprüche, bei der das sulfatierte Polysaccharid im Mittel wenigstens 0,1 Sulfatgruppe für jeden Saccharidrest des Polysaccharides umfaßt.

7. Verwendung nach Anspruch 6, bei der das sulfatierte Polysaccharid im Mittel wenigstens 1 Sulfatgruppe für jeden Saccharidrest des Polysaccharides umfaßt.

8. Verwendung nach einem der vorangehenden Ansprüche, bei der das sulfatierte Polysaccharid ein mittleres Molekulargewicht im Bereich von 25.000 bis 250.000 hat.

9. Verwendung nach einem der vorangehenden Ansprüche, bei der das sulfatierte Polysaccharid in Form eines gewebten, ungewebten oder geknüpften Stoffes vorliegt.

10. Verwendung nach einem der vorangehenden Ansprüche, bei der das sulfatierte Polysaccharid in Form eines festen Komplexes mit Collagen vorliegt.

11. Verwendung nach einem der vorangehenden Ansprüche, bei der das sulfatierte Polysaccharid in Wasser zu einem Ausmaß von wenigstens 10 g/L bei 25°C löslich ist.

## Revendications

1. Utilisation d'un polysaccharide sulfaté chimiquement choisi parmi les dérivés cellulosiques sulfatés et les polysaccharides polyanioniques chimiquement sulfatés pour la préparation d'une composition pour le traitement d'une plaie chronique.

2. Utilisation selon la revendication 1, dans laquelle on choisit le polysaccharide sulfaté parmi les hydroxyéthylcellulose sulfatée, carboxyméthylcellulose sulfatée et cellulose régénérée oxydée sulfatée.

3. Utilisation selon la revendication 2, dans laquelle le polysaccharide sulfaté est la cellulose régénérée oxydée sulfatée.

4. Utilisation selon la revendication 1, dans laquelle on choisit le polysaccharide sulfaté parmi les alginates sulfatés, les pectines sulfatées et l'acide hyaluronique sulfaté.

5. Utilisation selon la revendication 4, dans laquelle le polysaccharide sulfaté est un alginate sulfaté.

6. Utilisation selon l'une quelconque des revendications précédentes, dans laquelle le polysaccharide sulfaté comprend une moyenne d'au moins 0,1 groupe sulfate pour chaque résidu saccharide du polysaccharide.

7. Utilisation selon la revendication 6, dans laquelle le polysaccharide sulfaté comprend une moyenne d'au moins 1 groupe sulfate pour chaque résidu saccharide du polysaccharide.

8. Utilisation selon l'une quelconque des revendications précédentes, dans laquelle le polysaccharide sulfaté a une masse moléculaire moyenne dans la gamme de 25.000 à 250.000.

9. Utilisation selon l'une quelconque des revendications précédentes, dans laquelle le polysaccharide sulfaté est sous forme d'un textile tissé, non tissé ou tricoté.

10. Utilisation selon l'une quelconque des revendications précédentes, dans laquelle le polysaccharide sulfaté est sous forme d'un complexe solide avec du collagène.

11. Utilisation selon l'une quelconque des revendications précédentes, dans laquelle le polysaccharide sulfaté est soluble dans l'eau à un degré d'au moins 10 g/l à 25°C.
